# EUROPEAN PATENT SPECIFICATION

(11) **EP 4 199 846 B1**
(45) Date of publication and mention of the grant of the patent: **29.04.2026**
(21) Application number: 21801692.1
(22) Date of filing: 24.09.2021
(51) Int. Cl.: A61B 34/20, A61B 17/34

(54) **IMPROVED MAGNETIC TRAJECTORY PREDICTION AND POSITION IDENTIFICATION**
VERBESSERTE MAGNETISCHE TRAJEKTORIENVORHERSAGE UND -POSITIONSIDENTIFIZIERUNG
PRÉDICTION DE TRAJECTOIRE MAGNÉTIQUE ET IDENTIFICATION DE POSITION AMÉLIORÉES

(30) Priority: 25.09.2020 US 202063083624 P
(43) Date of publication of application: 28.06.2023
(73) Proprietor: Bard Access Systems, Inc., Salt Lake City, UT 84116 (US)
(72) Inventor: TRAN, Huy Ngoc, Riverton, UT 84065 (US); DIAMOND, Jordan P., Salt Lake City, UT 84105 (US); WESTWOOD, Paul T., Kaysville, UT 84037 (US); LENGYEL, Andrew J., Sandy, UT 84093 (US); TEERLINK, Trevor, Salt Lake City, UT 84106 (US); MISENER, Anthony K., Bountiful, UT 84010 (US)
(74) Representative: Hoffmann Eitle
(86) International application number: PCT/US2021/052066
(87) International publication number: WO 2022/067108

(56) References cited:
- EP-A1- 0 153 021
- US-A1- 2017 347 914
- US-A1- 2018 116 551
- US-A1- 2018 310 955
- US-B2- 9 950 139

## Description

### PRIORITY

This application claims priority to U.S. Provisional Application No. 63/083,624, filed September 25, 2020.

### SUMMARY

Briefly summarized, embodiments disclosed herein are directed to trackable medical devices with improved magnetic location or orientation identification, and improved trajectory prediction. The invention is defined by the appended claims set.

Magnetic based tracking systems, such as those used to track medical devices, needles, catheters, or the like, rely on detecting magnetic field of a magnetizable component associated with the trackable medical device. In an embodiment, the trackable medical device can include a catheter placement system having a needle that can be magnetized to provide a magnetic field. The magnetic tracking system can be configured to detect a strength or signature of the magnetic field to determine a location, orientation, or trajectory of a needle of the catheter placement system. US 2018/310955 A1 discloses a magnetizing system for magnetizing a needle of a medical device US 9950139 B2 discloses a medical device comprising a needly. EP 0153021 A1 discloses an IUD having a magnetized portion and an extractor device for removing the IUD from a patient.

However, additional components of the catheter placement system that are also formed of a magnetizable materials can provide additional magnetic fields. The strength or signature of these secondary magnetic fields can obscure or interfere with the strength or signature of the first magnetic field, inhibiting the performance of the tracking system to accurately determine the location, orientation, or trajectory of the needle. Disclosed herein are medical devices including a needle formed of a magnetizable material, and additional components formed of a non-magnetizable material to inhibit obstruction or interference of the first magnetic field from the needle.

However, replacing components formed of magnetizable materials, with the same components formed of non-magnetizable materials presents additional problems. Non-magnetizable materials present different mechanical properties, and as such, the performance of these components can vary greatly leading to reduced function or failure of the medical device overall. Also disclosed herein are components formed of non-magnetizable materials and configured to provide the same mechanical performance as components formed of the magnetizable materials.

Disclosed herein is a trackable catheter placement system including, a needle formed of a magnetizable material and magnetized to produce a magnetic field having one or both of a magnetic field strength and a magnetic field signature detectable by a sensor of a tracking system, and a spring formed of a non-magnetizable material and configured to display the same mechanical performance properties as a spring formed of the magnetizable material.

The non-magnetizable material includes an alloy including copper and beryllium, or a silver coated copper beryllium alloy, The magnetizable material includes 17-7 precipitation hardened stainless steel. In some embodiments, the spring includes a wire core diameter of 0.0113 ± 0.001 inches (0.287 ± 0.003 mm). In some embodiments, the spring includes between 30 and 37 active coils. In some embodiments, the spring includes a solid length of between 0.45 inches and 0.49 inches (between 11mm and 12 mm). In some embodiments, the spring includes a coil pitch angle of between 12 degrees and 16 degrees.

In some embodiments, the spring includes a spring diameter of 0.205 ± 0.005 inches (5.21 ± 0.1mm). In some embodiments, the spring includes 3 dead coils at a distal end and 2 dead coils at a proximal end. In some embodiments, the spring includes a distal flared diameter of 0.215 inches (5.46 mm). In some embodiments, the tracking system includes a passive magnetic tracking system configured to detect a magnetic field strength of the needle. In some embodiments, the tracking system includes an electro-magnetic tracking system configured to detect a magnetic field signature of the needle.

According to claim 1, there is provided method of manufacturing a trackable medical device including, providing a medical device including a needle extending from a distal end of a body and a non-magnetizable spring disposed within the body, the needle configured to access a vasculature of a patient and formed of one of 304-stainless steel or 17-7 precipitation hardened stainless steel, the spring including copper and beryllium, placing a portion of the medical device within a magnetizer that includes a magnetic element, magnetizing the needle, and providing a first magnetic signal from the needle.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a perspective view of an exemplary medical device, in accordance with embodiments disclosed herein.
FIG. 1B shows an exploded view of an exemplary medical device, in accordance with embodiments disclosed herein.
FIG. 2A shows an exemplary tracking system used to track a medical device including magnetizable components, in accordance with embodiments disclosed herein.
FIG. 2B shows an exemplary tracking system used to track a medical device including non-magnetizable components, in accordance with embodiments disclosed herein.
FIG. 3 shows configuration details of an exemplary spring, in accordance with embodiments disclosed herein.
FIG. 4A shows a perspective view of an exemplary magnetizer that can magnetize the medical device of FIG. 1A, in accordance with embodiments disclosed herein.
FIG. 4B shows an exploded view the magnetizer device of FIG. 4A, in accordance with embodiments disclosed herein.
FIG. 5A shows a perspective view of a sensor of a tracking system including the *x*-axis and the *z*-axis, in accordance with embodiments disclosed herein.
FIG. 5B shows a side view of a sensor of a tracking system including the *z*-axis and the *y*-axis, in accordance with embodiments disclosed herein.
FIGS. 6A-6D show bar charts of the results from testing exemplary medical devices, in accordance with embodiments disclosed herein.

### DESCRIPTION

Before some particular embodiments are disclosed in greater detail, it should be understood that the particular embodiments disclosed herein do not limit the scope of the appended claim. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a needle disclosed herein includes a portion of the needle intended to be near a clinician when the needle is used on a patient. Likewise, a "proximal length" of, for example, the needle includes a length of the needle intended to be near the clinician when the needle is used on the patient. A "proximal end" of, for example, the needle includes an end of the needle intended to be near the clinician when the needle is used on the patient. The proximal portion, the proximal end portion, or the proximal length of the needle can include the proximal end of the needle; however, the proximal portion, the proximal end portion, or the proximal length of the needle need not include the proximal end of the needle. That is, unless context suggests otherwise, the proximal portion, the proximal end portion, or the proximal length of the needle is not a terminal portion or terminal length of the needle.

With respect to "distal," a "distal portion" or a "distal end portion" of, for example, a needle disclosed herein includes a portion of the needle intended to be near or in a patient when the needle is used on the patient. Likewise, a "distal length" of, for example, the needle includes a length of the needle intended to be near or in the patient when the needle is used on the patient. A "distal end" of, for example, the needle includes an end of the needle intended to be near or in the patient when the needle is used on the patient. The distal portion, the distal end portion, or the distal length of the needle can include the distal end of the needle; however, the distal portion, the distal end portion, or the distal length of the needle need not include the distal end of the needle. That is, unless context suggests otherwise, the distal portion, the distal end portion, or the distal length of the needle is not a terminal portion or terminal length of the needle.

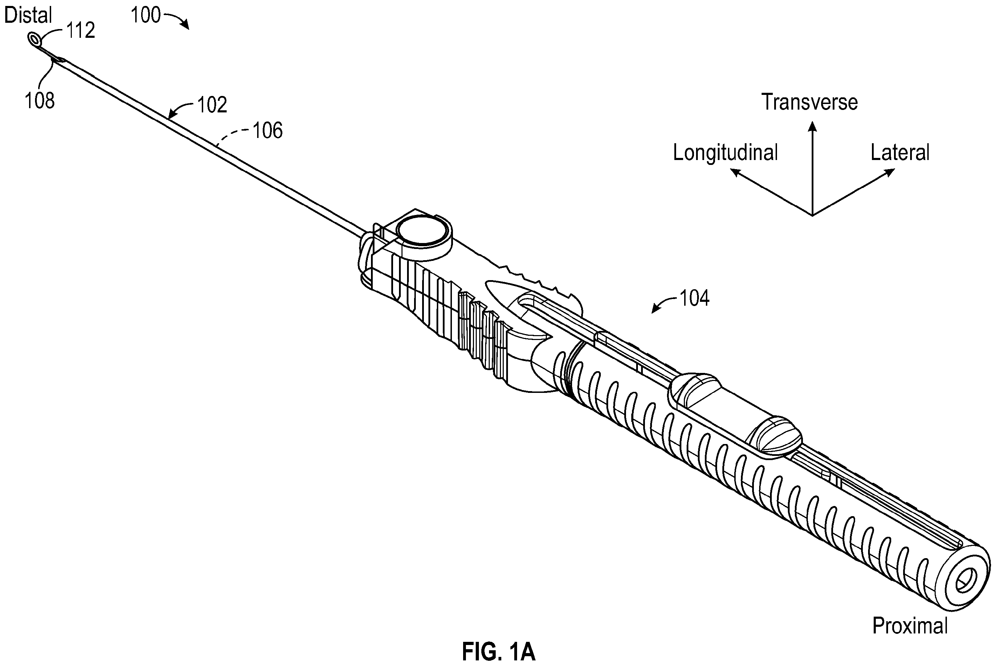

To assist in the description of embodiments described herein, and as shown in FIG. 1A, a longitudinal axis extends substantially parallel to an axial length of a needle 102. A lateral axis extends normal to the longitudinal axis, and a transverse axis extends normal to both the longitudinal and lateral axes.

As used herein, a "magnetic strength" is defined as an absolute measurement of a magnetic field strength. As used herein, a "magnetic signature" is defined as a difference in one or more parameters that define an electro-magnetic field. For example a wave length, wave frequency, wave amplitude, combinations thereof, or the like.

Unless defined otherwise, all technical and scientific terms used herein have the same meaning as commonly understood by those of ordinary skill in the art.

FIGS. 1A-1B show details of an exemplary medical device 100 generally including an elongate cannula or needle 102 extending along a longitudinal axis and supported at a proximal end by a hub, housing, or elongate body 104. In an embodiment, the medical device 100 can include a catheter placement system, needle, cannula, trocar, stylet, guidewire, or similar elongate medical device including a portion configured to be inserted subcutaneously into a patient. In an embodiment, the catheter placement system can be an ACCUCATH^{™} catheter placement system, or the like. As used herein, an exemplary medical device 100 may also be referred to as a catheter placement system 100. Similarly, the body 104 is also not intended to be limiting and can also include various handles, housings, connectors, extension legs, or similar support or connecting structures. It will be appreciated that, as used herein, the term "medical device" is exemplary and not intended to be limiting, and embodiments described herein can be used for any device that can be tracked by a magnetic tracking system. For example, trackable devices used within the construction industry, surveying, or the like.

In an embodiment, the needle 102 can define a lumen 106 extending along the longitudinal axis and providing fluid communication between a distal tip 108 of the needle 102 and a proximal end 110 of the needle 102. In an embodiment, the needle 102 or cannula can define a sharpened distal tip 108. In an embodiment, the medical device 100 can include a catheter 120, sheath, or similar tubular device disposed on an outer surface of the needle 102. The catheter 120 can be supported by a catheter hub 122. The catheter 120 can be selectively detachable from the needle 102. In an embodiment, the medical device 100 can include a second elongate medical device, e.g. a guidewire 112, or the like, extending through the needle lumen 106. In an embodiment, the second elongate medical device can be formed of a non-magnetizable material, as described herein.

In an embodiment, the needle 102 can be slidably engaged with the body 104 along the longitudinal axis. In an embodiment, the medical device 100 can include a needle retraction system 130. The needle retraction system 130 can be configured to withdraw the needle 102 proximally into the body 104 after the catheter 120 has been placed successfully. Advantageously, the needle 102 can be withdrawn into the body 104 to mitigate accidental needle stick injuries and/or mitigate contamination of fluids, e.g. blood, disposed thereon.

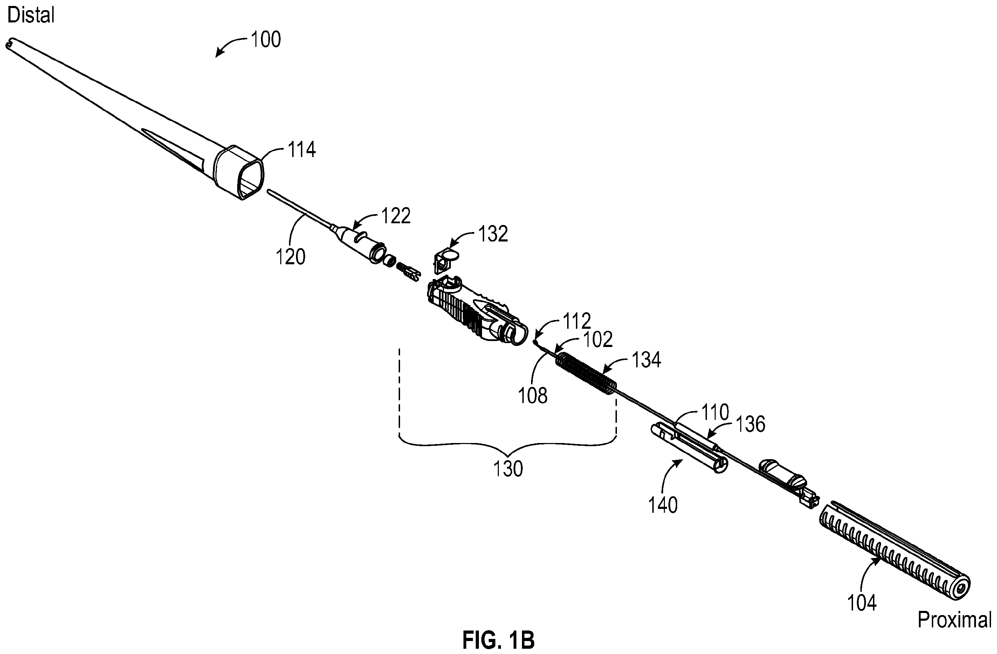

The needle retraction system 130 can include an actuator 132 and a biasing member 134, e.g. compression spring or the like, disposed within a portion of the body 104. The biasing member 134 can be coupled to the needle 102 and can bias the needle 102 towards a retracted position, disposed within the body 104. The actuator 132 can be configured to retain the needle 102 in an extended position, where a portion of the needle 102 is disposed distally of a distal end of the housing 104, e.g. as shown in FIG. 1. Actuating the actuator 132 can allow the biasing member 134 to urge the needle towards the retracted position within the body 104. Further details of catheter placement systems including needle retraction systems can be found in U.S. Patent No. 5,865,806; U.S. Patent No. 5,911,705; U.S. Patent No. 8,728,035; U.S. Patent No. 9,162,037; and U.S. Patent No. 10,220,191.

In an embodiment, the medical device 100 can include a blood flash indicator 136 in fluid communication with the needle. The blood flash indicator 136 can be configured to indicate when a distal tip of the needle 108 has accessed a vasculature of a patient. In an embodiment, the medical device 100 can include a guidewire advancement assembly 140 slidably engaged with the body 104 and configured to selectively advance a guidewire 112 through a needle lumen 106 and into a vasculature of the patient. In an embodiment, the medical device 100 can include a cap 114 disposed over the needle 102 extending from the distal end of the body 104, and selectively removable therefrom and configured to protect the needle 102 during storage or transport.

In an embodiment, a portion of the medical device 100 can be magnetized, e.g. needle 102, and used with various tracking systems that employ one or more tracking modalities. Exemplary modalities can include ultrasound, passive ("permanent") magnetic tracking, electro-magnetic tracking, combinations thereof, or the like.

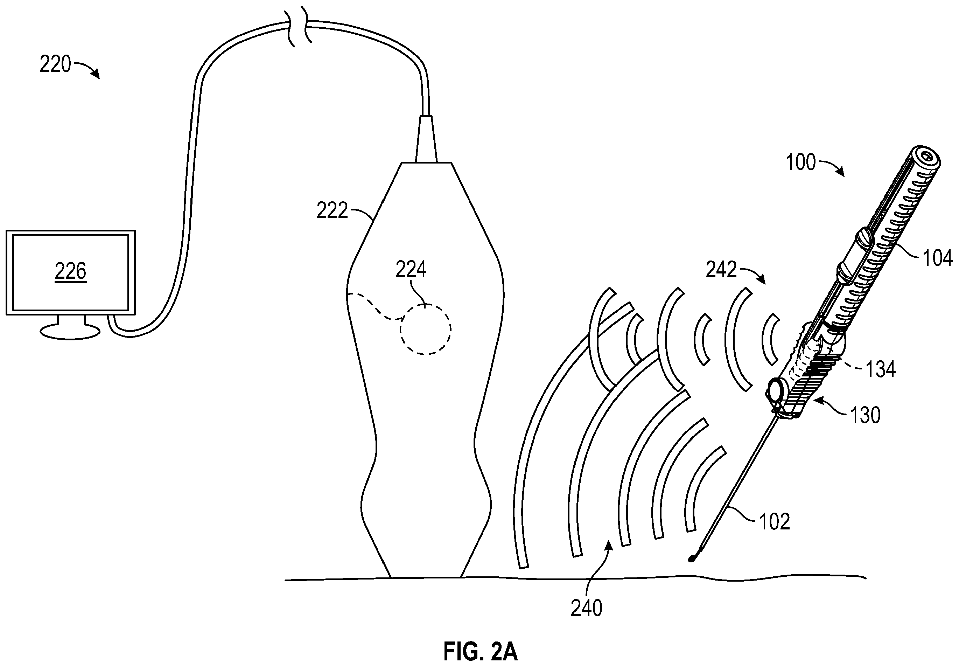

FIGS. 2A-2B show details of an exemplary tracking system 220. The tracking system 220 can generally include a probe 222 communicatively coupled to a console 226. The probe 222 can include an ultrasound transducer and one or more sensors 224. The ultrasound transducer can be configured to emit and receive acoustic signals to provide an image of a subcutaneous target location, e.g. a target vessel or the like. The one or more sensors 224 can be configured to detect one or more modalities, e.g. magnetic field, electro-magnetic field, or the like. In an embodiment, the tracking system 220 can utilize an ultrasound modality to image a subcutaneous target area, and also use a magnetic modality to track a position of the medical device 100 relative to the probe 222. The tracking system 220 can further include a console 226 having a display configured to show both the imaged subcutaneous target area, as well as the position of the medical device 100 relative to the target area.

The medical device 100, or a portion thereof, includes a magnetizable material for example a metal, alloy, composite, steel, stainless steel, 304-stainless steel, 17-7 Precipitation Hardened Stainless Steel ("17-7 PH SS"), or similar magnetizable material. The needle 102 of the medical device 100 is formed of a magnetizable material and can be magnetized to provide a passive ("permanent") magnetic field. In an embodiment, the medical device 100 can include one more components configured to produce an electro-magnetic field from the needle 102. As such, the magnetized medical device 100 creates a magnetic field that can be detected by the probe 222 of the tracking system 220. The tracking system 200 can detect and analyze the strength and/or signature of the magnetic field and determine a position, orientation, or trajectory of the medical device 100 relative to the tracking system 220.

Further details of such multi-modal tracking systems can be found, for example, in U.S. 8,388,541, U.S. 8,971,994, U.S. 9,492,097, U.S. 9,636,031, U.S. 10,238,418, U.S. 10,966,630, U.S. 11,027,101, U.S. 2018/0116551, U.S. 2018/0304043, U.S. 2019/0069877, U.S. 2019/0099108, U.S. 2020/0054858, U.S. 2020/0237255, and U.S. 2020/0345983.

With continued reference to FIG. 2A, an exemplary medical device can include one or more components which are separate from the magnetized needle 102, and also formed of a magnetizable material, e.g. steel. The one or more components can include springs, clips, needle safety clips, guidewire advancement assemblies, blood flash indicators, portions thereof, or similar structures that rely on steel or similar ferrous material to provide preferred mechanical properties and provide a desired performance of mechanical functions. These components, although may not be intentionally magnetized, may still provide a second magnetic signal 242 that is different in strength and/or magnetic signature from the first magnetic signal 240 of the needle 102.

The second magnetic signal 242 of the components can obscure or interfere with the first magnetic signal 240 of the needle 102, making the first magnetic signal 240 harder to distinguish and reducing the accuracy of the tracking system 220 to predict a location, orientation, or trajectory of the needle 102.

In an embodiment, the second magnetic signal 242 can obscure the strength of the first magnetic signal 240 by providing a second magnetic signal 242 having a second magnetic field strength. This second magnetic field strength, or "background noise," can reduce the signal to noise ratio between the first magnetic signal 240 relative to the background noise making the first magnetic signal 240 harder to distinguish, or obscuring the first magnetic signal 240 altogether.

In an embodiment, the second magnetic signal 242 of the components can interfere with the signature of the first magnetic signal 240. For example, where the first magnetic signal 240 is provided as an electro-magnetic wave, having a distinct magnetic signature, the second magnetic signal 242 can interfere with the first magnetic signal 240 through constructive or destructive waves, or the like. The obstruction or interference of the first magnetic signal 240 by the second magnetic signal 242 can be termed as providing a "dirty" signal that can reduce the accuracy of the tracking system 220 in determining a location, orientation, or trajectory of the medical device 100.

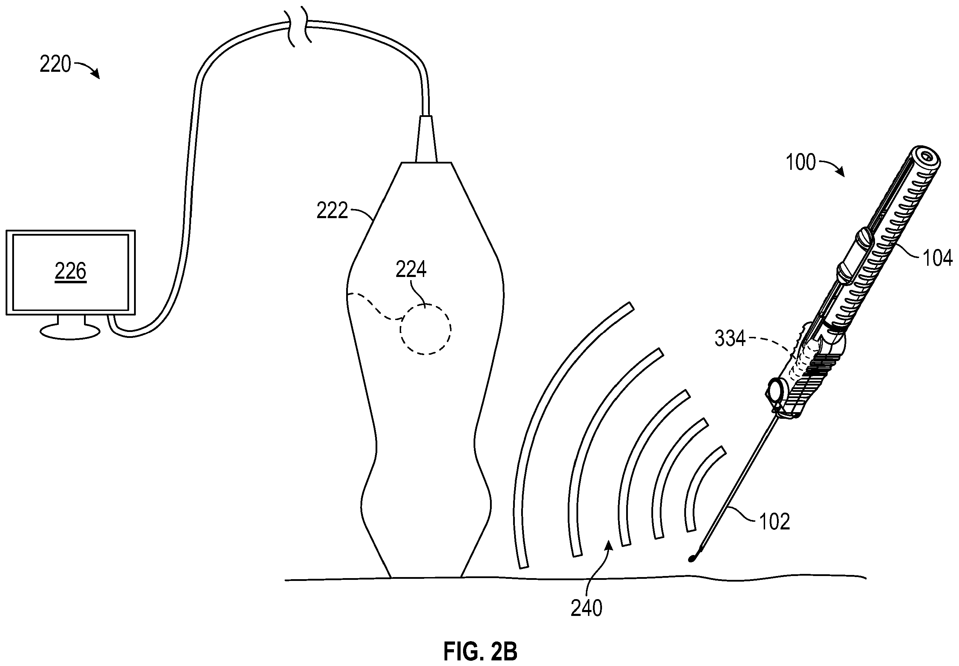

As shown, in FIG. 2B, the medical device 100 includes components formed of a non-magnetizable material, in particular non-magnetizable spring 334 including copper and beryllium. Exemplary non-magnetizable materials can include non-ferrous metals, alloys, plastics, polymers, copper, beryllium, copper beryllium alloys, silver coated copper beryllium, or the like. The non-magnetizable components 334 provide little or no magnetic signal and, as such, the medical device 100 provides only the first, "clean" magnetic signal 240, e.g. from the needle 102. The first magnetic signal 240 is unobstructed and/or un-interfered by any secondary magnetic signals. The signal strength is discernable above a reduced background noise, i.e. a larger signal to noise ratio, and the signature of the signal is not interfered with. This increases the accuracy of tracking system 220 to track and predict the location, orientation or trajectory of the medical device 100.

It is important to note, however, that non-magnetizable metals, alloys, plastics, polymers, copper, beryllium, copper beryllium alloys, silver coated copper beryllium, or the like display different mechanical properties from that of magnetizable materials, e.g. steel, stainless steel, 304-stainless steel, 17-7 PH SS, or the like. Exemplary mechanical properties can include, density, minimum tensile strength, modulus of elasticity (stretch), modulus of torsion (compression), sheer strength, malleability, combinations thereof, or the like. As such the mechanical performance of the non-magnetizable components can differ substantially from non-magnetizable components leading to reduced performance or failure of the medical device.

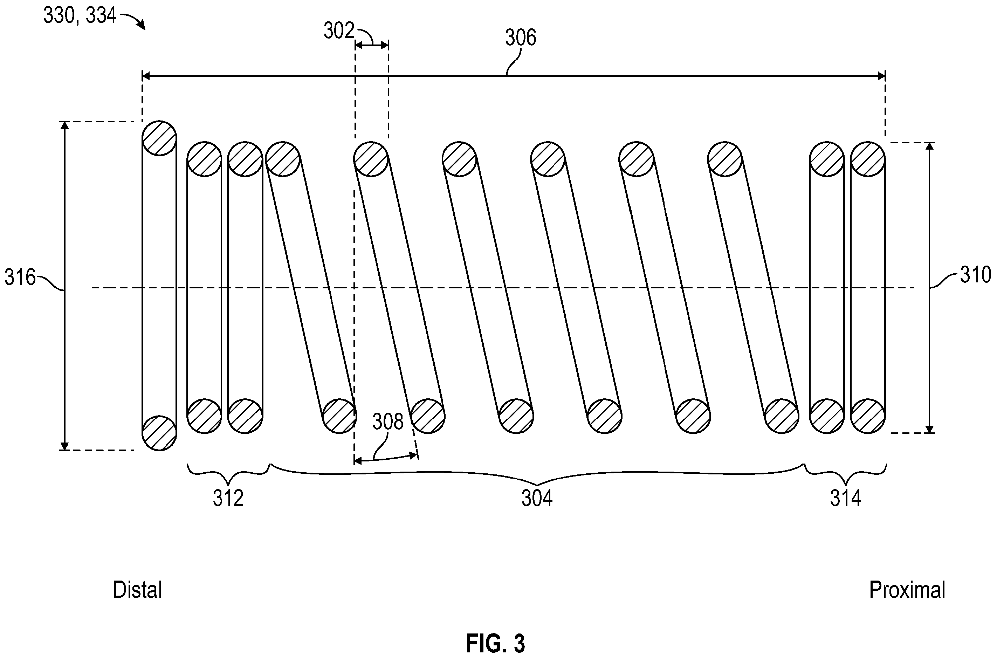

FIG. 3 and Table 1 below show configuration details of a spring 330 formed of a magnetizable material, e.g. 304-stainless steel, and configuration details of a spring 334 formed of a non-magnetizable material, e.g. silver coated copper beryllium. For example, Table 1 below shows configuration details for an exemplary magnetizable spring 330 and an exemplary non-magnetizable spring 334. The non-magnetizable spring 334 can be formed of silver coated copper beryllium and provide the same mechanical performance as the magnetizable spring 330 formed of 304-stainless steel. Table 1 below compares the differences in spring design between a magnetizable spring 330 and a non-magnetizable spring 334.

**Table 1: Example Difference in Spring Configuration**

| **Example Difference in Spring Design** | | | |
|---|---|---|---|
| | Spring Material | Magnetizable Spring 330 | Non-magnetizable Spring 334 |
| 302 | Wire Core-Diameter [in] | 0.010 ± 0.001 (0.025 ± 0.03 mm) | 0.0113 ± 0.001 (0.287 ± 0.003 mm) |
| 304 | Active Coils [#] | 44 | 33.5 |
| 306 | Solid Length [in] | 0.510 (13.0 mm) | 0.472 (12.0 mm) |
| 308 | Pitch Angle [deg] | 11.35 | 14.2 |
| 310 | Spring Diameter [in] | 0.195 ± 0.003 (4.95 ± 0.08 mm) | 0.205 ± 0.005 (5.21 ± 0.1mm) |
| 312 | Dead Coils at Distal end [#] | 4 | 3 |
| 314 | Dead Coils at Proximal end [#] | 2 | 2 |
| 316 | Distal Flared Diameter [in] | 0.212 (5.38 mm) | 0.215 (5.46 mm) |

FIG. 3 shows an exemplary spring indicating the configuration measurements listed in Table 1. The wire core diameter parameter (in.) 302 is the diameter of the wire that forms the spring 330, 334. The active coils parameter (#) 304, is the number of active coils within spring 330, 334. As shown, these active coils 304 are disposed between dead coils 312, 314 disposed at either end of the spring 330, 334. However, it will be appreciated that the spring 330, 334 can include one or more active coils 304 at one of the proximal end or distal end, or can include one or more dead coils 312, 314 disposed at a mid-point of the spring 330, 334. A solid length parameter (in.) 306, is the longitudinal length of the spring from a proximal end to a distal end. A pitch angle parameter (deg) 308, is the angle of the coil 304, 312, 314 relative to an axis that extends perpendicular to the longitudinal axis of the spring 330, 334. A spring diameter parameter (in.) 310 is the outer diameter of the spring extending along an axis that extends perpendicular to the longitudinal axis. The dead coils (#) at the distal end 312, and the dead coils (#) at the proximal end 314 parameters are the number of coils that extend substantially perpendicular to the longitudinal axis, and disposed at the distal end and proximal end, respectively. The distal flared diameter parameter (in.) 316 is the widest diameter of the spring extending along an axis that extends perpendicular to the longitudinal axis at one or both of the proximal end and the distal end.

Advantageously, the difference in configuration of the non-magnetizable spring 334 from that of the magnetizable spring 330 provides a spring that has the same mechanical performance with little or no change in the overall dimensions of the spring. For example, a difference in one of the number of active coils, a pitch angle of the coils, or the diameter of the wire-core, of the spring can modify a mechanical performance of the non-magnetizable spring 334 to be equal to that of a magnetizable spring 330 while maintaining a diameter or length of the spring as substantially the same. Advantageously, the non-magnetizable spring 334, configured as such can be used in existing designs of medical device 100 to provide the same mechanical performance without having to reconfigure the dimensions of the medical device 100 to accommodate a completely different spring.

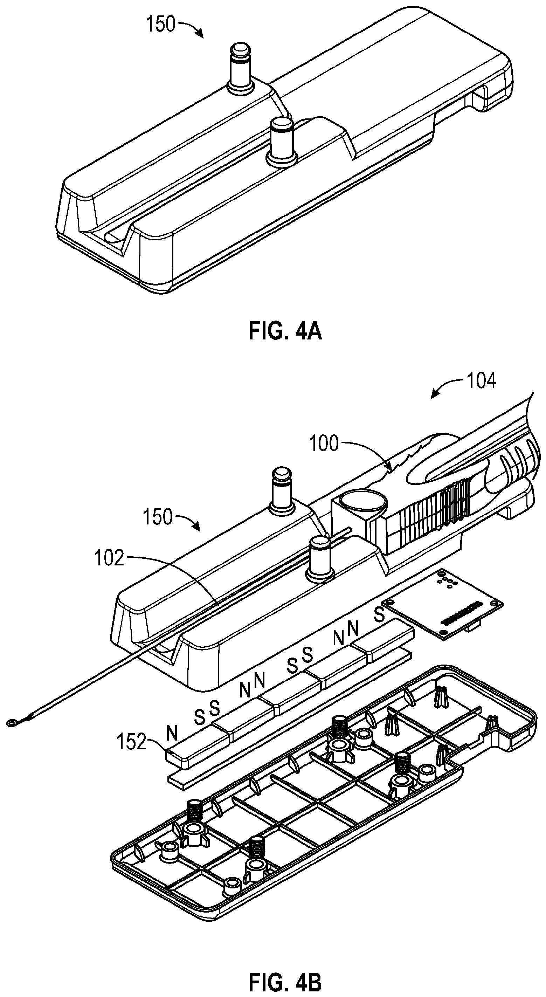

As shown in FIGS. 4A-4B, in an embodiment, an exemplary method of magnetizing the medical device 100 is provided. As shown in FIG. 4A, a magnetization device ("magnetizer") 150 is provided that includes one or more magnetic elements 152. The magnetic element 152 can include either permanent magnets, electro-magnets, or combinations thereof. The magnetization device 150 can be configured to align the medical device 100 relative to the magnetic elements 152 at a predetermined orientation. The magnetization device 150 then exposes the medical device 100, or a portion thereof, to the magnetic elements 152 to magnetize the medical device 100. Exposure of the medical device 100 to the magnetic elements 152 aligns the electrons of the metallic portions of the medical device 100 and imparts a magnetic field on the medical device 100. In an embodiment, the field lines of the magnetic field are aligned with an axis of the medical device 100. Further details of exemplary magnetizing devices can be found in U.S. Pub. No. 2018/0310955.

Advantageously, magnetizing the medical device 100 directly provides a simplified manufacture process, reducing complexity, and improving manufacturing speed and costs. This is relative to a medical device that includes a separate, permanent, magnetic material included with the medical device 100 to provide a magnetic field. For example, providing a needle 102 of a medical device 100 formed of a first material, e.g. 304 stainless steel, and including a permanent magnet formed of a second material, e.g. ferrite, requires increased complexity in combining the two contrasting materials into the one device. Further, the device requires aligning the magnetic field with the orientation of the needle 102. Similarly, forming a medical device 100 including an electromagnetic element requires yet further complexity in manufacturing and requires a power source.

As will be appreciated, one or more components of the medical device 100 can become magnetized when the medical device 100 is placed in proximity to the magnetizer 150. Since these additional components, e.g. magnetizable spring 134, are positioned and aligned differently relative to the magnetic elements 152 of the magnetizer 150, the secondary magnetic signal(s) 242 imparted on these one or more components can interfere with the primary magnetic signal 240 of the needle 102, as described herein.

In an embodiment, a medical device 100 is provided including a non-magnetizable spring 334 being formed of a copper beryllium alloy, silver coated copper beryllium, or combinations thereof, and configured as described in Table 1. Advantageously, the non-magnetizable spring 334 can be particularly resistant to being magnetized when the medical device 100 is exposed to the magnetizer 150, thereby reducing or eliminating any secondary magnetic signal 242 or "background noise" from the spring 334. Further, the spring 334 formed of copper, beryllium, or alloys thereof and configured as described in Table 1, can provide the same outer dimensions and the same mechanical performance as the magnetizable spring 134 formed of steel, stainless steel, 304 stainless steel, or the like. As such, the non-magnetizable spring 334 can be placed within the medical device 100 without any further redesign or reconfiguration of the medical device 100 reducing inventory and associated costs in manufacturing and logistics.

**Table 2: Composition comparison of 17-7 Stainless Steel and 304 Stainless Steel**

| **Chemical** | **17-7 Stainless Steel (%)** | **304 Stainless Steel (%)** |
|---|---|---|
| Chromium | 16.0 - 18.0 | 18.0 - 20.0 |
| Nickel | 6.50 - 7.75 | 8.0 - 10.5 |
| Aluminum | 0.75 - 1.50 | 0.00 |
| Manganese | up to 1.00 | up to 2.0 |
| Silicon | up to 1.00 | up to 0.75 |
| Carbon | up to 0.09 | up to 0.08 |
| Phosphorus | up to 0.04 | up to 0.045 |
| Sulfur | up to 0.03 | up to 0.03 |
| Nitrogen | 0.00 | up to 0.10 |
| Iron | makes up the remainder | makes up the remainder |

In an embodiment, the medical device 100 can include a needle 102 formed of 17-7 PH SS. Table 2 above compares the chemical composition of 17-7 Stainless Steel with that of 304 Stainless Steel. In an embodiment, the 17-7 stainless steel, as detailed in Table 2, is further treated using a precipitation hardening process. The precipitation hardening process generally includes applying a solution treatment that includes heating the 17-7 stainless steel to a relatively high temperature and treating with a solution. This is followed by a quenching treatment which includes rapidly cooling the solution-soaked metal. This is followed by an aging process which includes heating the metal to a relative medium temperature followed by rapid cooling. The precipitation hardening process can be performed in a vacuum, or an inert atmosphere, and can include temperatures ranging from between 900 degrees and 1150 degrees Fahrenheit (between 480°C and 620°C). However, it will be appreciated that higher and lower temperatures are also contemplated. In an embodiment, the process can range in length time from one to several hours, depending on the exact material and characteristics desired, although shorter and longer times are also contemplated. The precipitation hardening process of 17-7 stainless steel provides 17-7 PH SS which includes uniformly dispersed particles within the metal's grain structure. This can hinder motion of the particles and thereby providing improved mechanical strength properties.

The 17-7 PH SS can display comparable, or improved, mechanical and corrosion resistant properties to that of standard 304 stainless steel ("304 SS"). Further, 17-7 PH SS can demonstrate improved magnetic permeability allowing the needle 102 of the medical device 100 to develop a stronger magnetic field, relative to devices formed of 304 stainless steel, when subjected to the same magnetization methods using the magnetizer 150, as described herein.

Advantageously, devices formed of 17-7 PH SS provide surprisingly improved tracking properties, relative to similar devices formed of 304 stainless steel. As such, medical devices formed of 17-7 PH SS can provide improved drop-out distance of approximately double the distance, an improved pairing distance of approximately double the distance, an improved true position vs. calculated position by reducing the error distance by up to half, or an improved immunity to magnetic interference. The improved immunity to magnetic interference can be shown by reducing the error distance from between 34% and 57%, as seen in devices formed of 304 stainless steel, down to less than 3%, as seen in similar devices formed from 17-7 PH SS, as described in more detail herein. (*See* Table 4). Further, there was less variation in magnetic field strength between individual needles formed of 17-7 PH SS when subjected to the same processes of magnetization. Further, needles formed of 17-7 PH SS were more resistant to demagnetization. Worded differently, the strength of magnetic fields were more reliable. As such, in an embodiment, a medical device 100 is provided including a needle 102 formed of 17-7 PH SS and a spring 334 formed of copper, beryllium, or alloys thereof and configured as detailed in Table 1. As such the medical device 100 can be magnetized by a magnetizer 150 to impart a strong and stable magnetic signal 240 on the needle 102 and minimizing any secondary magnetic signals 242 being imparted on the spring 334. This provides a high signal-to-noise ratio between the primary magnetic signal 240 and the secondary magnetic signal 242 improving accuracy and reliability of tracking the needle 102 using multi-modal tracking systems 220, as described herein.

This invention is further illustrated by the following exemplary experiment(s), which are not to be construed in any way as imposing limitations upon the scope thereof. On the contrary, it is to be clearly understood that resort may be had to various other embodiments, modifications, and equivalents thereof which, after reading the description herein, may suggest themselves to those skilled in the art.

### Experiment 1

This exemplary experiment shows superiority in the magnetic tracking properties of exemplary needles formed of 17-7 PH SS, compared with exemplary needles formed of 304 stainless steel, when magnetized by proximity to a magnetic source, as disclosed herein.

### Scope

These exemplary test results illustrate a difference in magnetic tracking performance between three exemplary magnetized needles.

### Materials

The three exemplary needle types that were tested include A) a 21G needle formed of 304 stainless steel; B) an 18G needle formed of 304 stainless steel; and C) an 18G needle formed of 17-7 PH SS. The specifications of the three types of needles used in the experiment are set forth below in Table 3:

**Table 3: Specifications of three types of needles used in the experiment**

| **Specifications** | **Needle A** | **Needle B** | **Needle C** |
|---|---|---|---|
| Gauge (G) | 21G | 18G | 18G |
| Material | 304 Stainless Steel | 304 Stainless Steel | 17-7 PH SS |

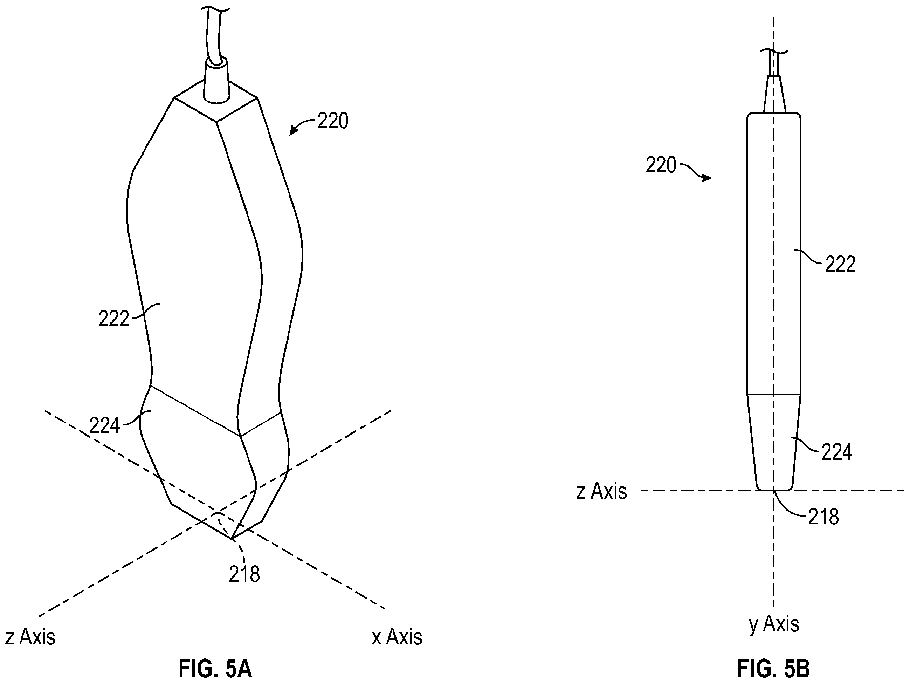

The tracking system 220 includes a probe 222 and a magnetic sensor 224 configured for detecting a magnetic field. The magnetic tracking properties were quantified based on the position of the test needle 102 relative to the sensor 224 in three-dimensional space. FIGS. 5A-5B show the *x*, *y*, and *z* - axes as defined in three dimensional space relative to the sensor 224 of the tracking system 220. The *x*, and *z* - axes extend horizontally and extend normally relative to each other, the *y* axis extends vertically relative to the *x*, *z* - axes. The *x*, *y*, and *z* - axes intersect at a center point 218 where the sensor 224 is located.

### Methods

The medical device(s) 100 to be tested included three separate needles 102 of each needle type A, B, C (*See* Table 3), i.e. needle A.1, A.2, A.3, B.1, B.2 etc. for a total of nine needles. As used herein a "true" distance is defined as a distance that is directly measured in three-dimensional space. As used herein a "calculated distance" is defined as a distance that is measured by a tracking system 220, such as those described herein, in three-dimensional space.

**Test 1) Drop-Out Distance:** As used herein, a drop-out distance is the maximum calculated distance between the magnetized needle 102 and the sensor 224 before the sensor 224 can no longer detect the presence of the needle 102. The test needle 102 was paired with the tracking system 220 and then moved away from the sensor 224 until the tracking system 220 failed to detect the needle 102, i.e. became unpaired. The last known position of the needle 102 was then recorded from the tracking system 220.

**Test 2) Pairing Distance:** As used herein, a pairing distance is the maximum true distance between the needle 102 and the sensor 224, at which the tracking system 220 can detect the presence of the needle 102, i.e. a successful pairing. The test needle(s) 102 were positioned at a predetermined location as defined by the *x*, *y*, and *z* - axes, and a successful pairing at the location was recorded. The needle 102 was then placed at different predetermined locations that were progressively further from the sensor 224, until the tracking system 220 could no longer pair with the needle 102. The last known successful pairing distance for each needle 102 was recorded.

**Test 3) True position vs. Calculated position:** As used herein, the true position vs. calculated position is defined as a comparison between the true location of the needle 102, as measured by true distance in three-dimensional space, compared with the calculated position of the needle 102, as measured by the tracking system 220. Initially the needle 102 is placed at a predetermined location, as defined by the *x, y,* and *z* - axes, and paired with the tracking system 220. The true position, as defined by the *x*, *y*, *z* -axes is then compared with the calculated position as defined by the tracking system 220 and the differences recorded.

**Test 4) Immunity to Magnetic Interference:** As used herein, immunity is defined as changes induced by the presence of a magnetic interference source. The changes measured were differences between the true position vs. the calculated position. The needle 102 was paired with the tracking device 220 and positioned at a predetermined location, as defined by the *x*, *y*, and *z* - axes. The true location and the calculated location were recorded without a magnetic interference source present. A constant source of magnetic interference (e.g. a permanent magnet) was introduced, and the difference between the true position vs. the calculated position was re-recorded and compared. In an embodiment, the magnetic interference source was positioned further from the sensor 224 than the needle 102.

### Data

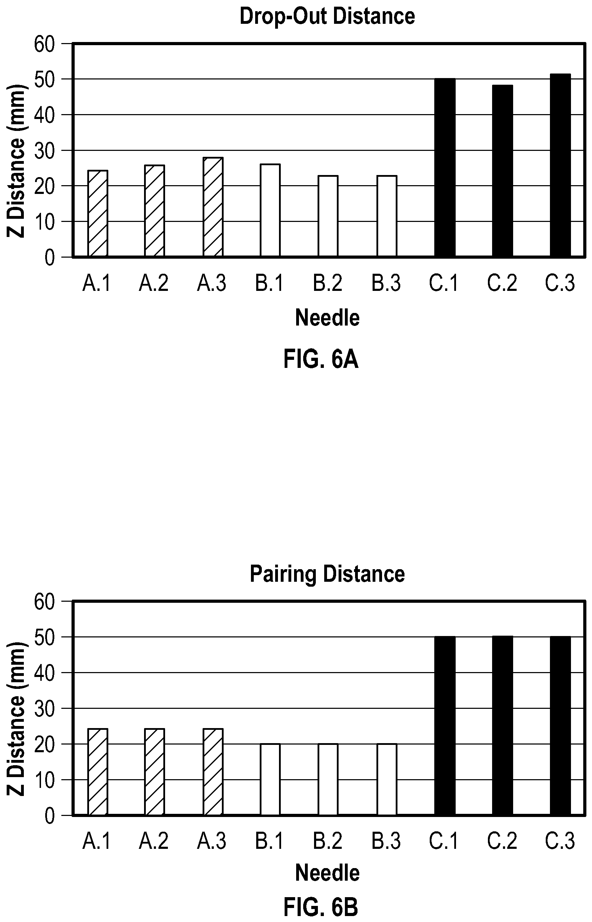

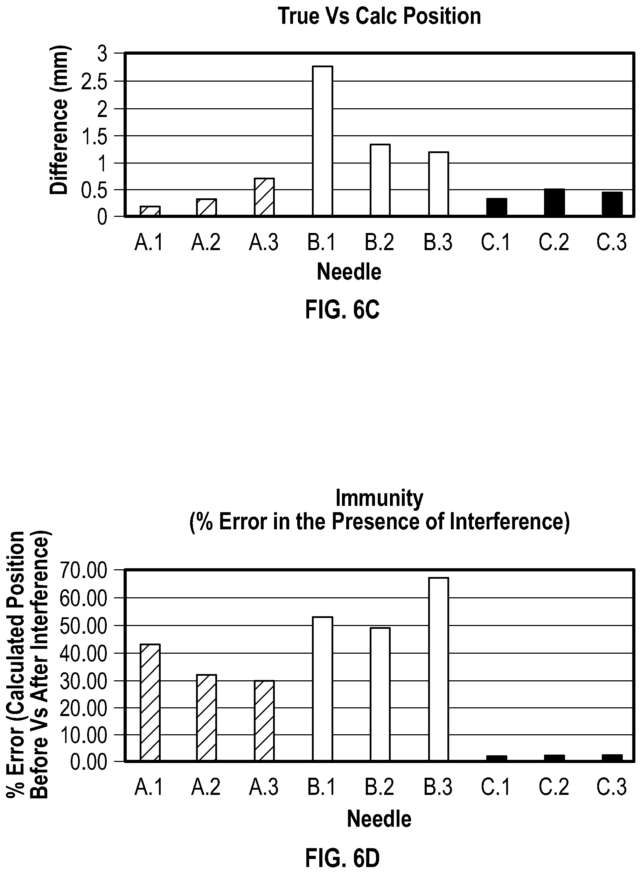

FIGS. 6A-6D show bar charts illustrating the data collected. The data is from a total of 9 total needles with 3 needles of each type, as described herein. FIG. 6A shows the drop-out distance for each needle. FIG. 6B shows the averaged pairing distance for each needle. FIG. 6C shows the difference between the true position and the calculated position for each needle. FIG. 6D shows a percentage change in error in calculated position in the presence of a magnetic interference source. It is important to note that the data presented herein is exemplary only and should not be considered as limiting to the scope of the invention.

### Results

Advantageously, it was found that needles formed of 17-7 PH SS, e.g. needle C, displayed a drop out distance and a pairing distance that was substantially double the drop out distance and pairing distance of needles formed of 304 SS, e.g. needle A and needle B. Further, the immunity of the 17-7 PH SS needles, needle C, to an interference source was improved by between 15 to 25 times. As such the calculated position of the needle varied less than 3% in the presence of the magnetic interference source. This compares with a change of between 34% and 57% of the calculated position for the 304 SS needles in the presence of the same magnetic interference source. The results of the exemplary tests are shown below in Table 4:

**Table 4: Results of the exemplary tests**

| **Tests** | | **Needle A** | **Needle B** | **Needle C** |
|---|---|---|---|---|
| **Drop-Out (mm)** | | 27.0 | 23.9 | 50.3 |
| **Pairing (mm)** | | 25.0 | 20.0 | 50.0 |
| **Immunity** | **(mm)** | 7.2 | 12.9 | 0.5 |
| | **% error** | 34.1 | 56.4 | 2.5 |
| **True Vs Calc. (mm)** | | 0.4 | 1.7 | 0.4 |

Based on these exemplary results, needles formed of 17-7 PH SS outperforms needles formed of 304 stainless steel in the measured aspects of magnetic based tracking. Overall, the needles formed of 17-7 PH SS offer a greater window of use when used with magnetic tracking systems, and are considerably less affected by magnetic interference.

While some particular embodiments have been disclosed herein, and while the particular embodiments have been disclosed in some detail, it is not the intention for the particular embodiments to limit the scope of the claim. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the claims.

## Claims

1. A method of manufacturing a trackable medical device, comprising:
providing a medical device including a needle (102) extending from a distal end of a body and a spring (134) disposed within the body, the needle configured to access a vasculature of a patient and formed of one of 304-stainless steel or 17-7 precipitation hardened stainless steel;
placing a portion of the medical device within a magnetizer (150) that includes a magnetic element (152);
magnetizing the needle (102); and
providing a first magnetic signal from the needle (102)
**characterised in that**
the spring (134) is non-magnetizable and includes copper and beryllium.

## Patentansprüche

1. Verfahren zum Herstellen einer verfolgbaren medizinischen Vorrichtung, umfassend:
Bereitstellen einer medizinischen Vorrichtung, die eine Nadel (102) einschließt, die sich von einem distalen Ende eines Körpers erstreckt, und eine Feder (134), die innerhalb des Körpers angeordnet ist, wobei die Nadel ausgebildet ist, um in ein Gefäßsystem eines Patienten einzudringen, und aus einem von 304-Edelstahl oder ausscheidungsgehärtetem 17-7-Edelstahl gebildet ist;
Platzieren eines Abschnitts der medizinischen Vorrichtung innerhalb eines Magnetisierers (150), der ein magnetisches Element (152) einschließt;
Magnetisieren der Nadel (102); und
Bereitstellen eines ersten magnetischen Signals von der Nadel (102),
**dadurch gekennzeichnet, dass**
die Feder (134) nicht magnetisierbar ist und Kupfer und Beryllium einschließt.

## Revendications

1. Procédé de fabrication d'un dispositif médical traçable, comprenant :
la fourniture d'un dispositif médical incluant une aiguille (102) s'étendant à partir d'une extrémité distale d'un corps et un ressort (134) disposé à l'intérieur du corps, l'aiguille étant configurée pour accéder au système vasculaire d'un patient et formée en acier inoxydable 304 ou acier inoxydable trempé par précipitation 17-7 ;
le placement d'une portion du dispositif médical à l'intérieur d'un magnétiseur (150) qui inclut un élément magnétique (152) ;
la magnétisation de l'aiguille (102) ; et
la fourniture d'un premier signal magnétique provenant de l'aiguille (102) **caractérisé en ce que**
le ressort (134) est non magnétisable et inclut du cuivre et du béryllium.
